# EUROPEAN PATENT APPLICATION

(11) **EP 2 014 762 A1**
(43) Date of publication of application: **14.01.2009**
(21) Application number: 07741815.0
(22) Date of filing: 11.04.2007
(51) Int. Cl.: C12M 1/34

(54) **BIOLOGICAL INDICATOR AND METHOD FOR PRODUCING THE SAME**

(30) Priority: 11.04.2006 US 790815 P
(71) Applicant: NGK INSULATORS, LTD., Nagoya-shi, Aichi 467-8530 (JP)
(72) Inventor: HIROTA, Toshikazu, Nagoya-shi, Aichi 467-8530 (JP); KIRA, Shigeki, Nagoya-shi, Aichi 467-8530 (JP); IMANISHI, Yuuichiro, Nagoya-shi, Aichi 467-8530 (JP)
(74) Representative: Paget, Hugh Charles Edward
(86) International application number: PCT/JP2007/058379
(87) International publication number: WO 2007/119857

(57) **Abstract**

A biological indicator including a carrier and an indicator material, said indicator material adhering on the surface of the carrier, and having a contact portion being contact with the surface of the carrier and a non-contact portion being not in contact with the surface of the carrier, and wherein the carrier includes an adhering area having a adhering portion to which the indicator material adheres and a non-adhering area having an non-adhering portion to which the indicator material does not adhere.

## Description

### Technical Field

The present invention relates to a biological indicator used to measure ability of a sterilization unit or the like and a manufacturing method thereof.

### Technical Background

For the purpose of confirming and ensuring the effectiveness of an apparatus for carrying out disinfection, sterilization, bacteria elimination, and toxin degradation (hereinafter simply referred to as "sterilization or the like") of a medical device or a medical container, performance thereof is checked constantly or periodically. As a method for the performance check, recently, following method has been adopted: A biological indicator is provided in an arbitrary spot of an apparatus, a container, or the like and sterilization degree measured by biological indicator after processing such as sterilization is set as an indicator for carrying out performance check.

As a related conventional technique, for example, it is described in [Description of the Related Arts] of the Patent Document 1 that "Proof Plus", which is a commercially-available product of AMSCO Scientific, can be used as a biological indicator for evaluating sterilization effect of ethylene oxide gas. Moreover, it is described in [Description of the Related Arts] of the Patent Document 2 that "Attest^{™} Rapid Readout Biological Indicator" of 3M, which is a commercially-available product, is designed to evaluate effectiveness of a steam sterilization process or ethylene oxide sterilization process.

Patent Document 1: JP-A-10-201466
Patent Document 2: JP-A-2006-521818

### Disclosure of the Invention

However, the commercially-available biological indicators have been designed on the premise that effect in case gas such as steam or ethylene oxide is used to sterilize a target is evaluated. Therefore, effectiveness of disinfection, sterilization, bacteria elimination, or toxin degradation system using radiation for disinfection, sterilization, bacteria elimination, or toxin degradation (hereinafter referred to as "acting radiation"), which carries out disinfection, sterilization, bacteria elimination, or toxin degradation by irradiation facially to a target, cannot always be correctly evaluated by such biological indicators and moreover, there are cases where the evaluation results varied. That is, there has not been a biological indicator which can correctly measure the ability of a sterilization unit or the like which carries out sterilization or the like by surface irradiation of the acting radiation.

Moreover, the amount of materials which contaminate a target to be actually processed (sterilized) is generally quite small. Therefore, there has not been a biological indicator having enough sensitivity to measure if the quite small amount of the contaminant has been removed or not.

The present invention has been made in consideration of the problems of the conventional arts and is aimed at providing a biological indicator which can measure the ability of a sterilization unit or the like for carrying out sterilization or the like by surface irradiation of acting radiation correctly with high sensitivity. Moreover, the present invention aims at providing a biological indicator which can measure if a very small amount of contaminant has been removed or not with high sensitivity and can correctly measure the ability of a sterilization unit or the like. Further, the present invention aims at providing a manufacturing method of a biological indicator which enables to easily manufacture a biological indicator which can measure the ability of a sterilization unit or the like correctly with high sensitivity.

As the inventors of the present invention keenly studied to solve the above-mentioned problems, the inventors found out that in a conventional biological indicator having a structure by which an indicator bacterium is adhered on a surface of a carrier such as a sheet by being applied thereon, there are many cases where adjacent bacteria are applied and adhered in an overlapping manner. Therefore, there are cases where acting radiation is not directly irradiated to a bacterium in a position covered by another bacterium and this is highly possibly preventing evaluation of effectiveness. Therefore, the inventors found out that the above-mentioned problems can be solved by the configuration shown below to complete the present invention.

That is, according to the present invention, a biological indicator and a manufacturing method thereof shown below are provided.

[1] A biological indicator includes a carrier and an indicator material; said indicator material adheres on the surface of the carrier and has a contact portion being contact with the surface of the carrier and a non-contact portion being not in contact with the surface of the carrier, and the carrier includes an adhering area having a adhering portion to which the indicator material adheres and a non-adhering area having an non-adhering portion to which the indicator material does not adhere.

[2] The biological indicator according to [1] wherein all of the indicator materials exist within a range of 2 µm from the carrier surface.

[3] The biological indicator according to [1] or [2], wherein a plurality of the adhering areas exist as a domain.

[4] The biological indicator according to [3] wherein the shape of the plurality of adhering areas existing as a domain is approximately circular form and density of the indicator materials in each of the plurality of adhering areas becomes higher in an outer circumference portion and in the vicinity thereof of the adhering area.

[5] The biological indicator according to any one of [1] to [4], wherein the indicator material is a spore-forming bacterium

[6] The biological indicator according to [5] wherein at least a part of 90% or more of the spore-forming bacteria can be visually confirmed when seen from a direction perpendicular to the surface of the carrier.

[7] The biological indicator according to [6] wherein adjacent spore-forming bacteria adhere on the carrier surface without overlapping to each other.

[8] The biological indicator according to [6] or [7] wherein a plurality of the adhering areas exist as a domain and at the same time 1 to 100 spore-forming bacteria are included in one adhering area.

[9] The biological indicator according to any one of [5] to [8], wherein at least protein or polymer exists on the carrier surface.

[10] The biological indicator according to [9] wherein the protein is albumin.

[11] The biological indicator according to [9] wherein the polymer is carboxyl methyl cellulose or polyethylene glycol.

[12] The biological indicator according to any one of [5] to [11], wherein the number of spore-forming bacteria adhering on the carrier surface is between 5 × 10⁵ and 2 × 10⁶.

[13] The biological indicator according to any one of [1] to [4], wherein the indicator material is endotoxin.

[14] The biological indicator according to [13] wherein all of the indicator materials exist within a range of 100 nm from the carrier surface.

[15] The biological indicator according to any one of [1] to [14], wherein the carrier is an impermeable substrate through which fluid cannot permeate or a permeable substrate through which fluid can permeate.

[16] The biological indicator according to any one of [1] to [15], wherein the material constituting the carrier is one member selected from the group consisting of polystyrene, polypropylene, polyethylene terephthalate, or stainless.

[17] The biological indicator according to any one of [1] to [16] to be used to evaluate effectiveness of a disinfection, sterilization, bacteria elimination, or toxin degradation apparatus using radiation for disinfection, sterilization, bacteria elimination, or toxin degradation which is aspect irradiated to a target.

[18] A method for manufacturing a biological indicator according to any one of [1] to [17] including a step of discharging solution or dispersion fluid including an indicator material from a discharging unit as minute droplets by ink jet method so that the droplets are caused to fall on a carrier surface and thereafter drying the droplets which have fallen on the carrier surface.

[19] The manufacturing method of a biological indicator according to [18], wherein the quantity of a drop of the minute droplet is between 1 and 500 pl.

[20] The manufacturing method of a biological indicator according to [18] or [19], wherein the indicator material is a spore-forming bacterium and the dispersion fluid includes the spore-forming bacterium dispersed with the concentration of 1 × 10² to 1 × 10⁷/µl.

[21] The manufacturing method of a biological indicator according to [20], wherein the dispersion fluid is alcohol dispersion fluid in which the spore-forming bacteria are dispersed in an alcohol solvent.

[22] The manufacturing method of a biological indicator according to [21], wherein the alcohol solvent is alcohol aqueous solution having alcohol concentration of between 20 and 80% by mass.

[23] The manufacturing method of a biological indicator according to any one of [20] to [22], wherein the dispersion fluid is the spore-forming bacteria dispersed into the concentration of 1 ×10³ to 1 × 10⁷/µl and the quantity of a drop of the minute droplet is between 50 and 200 pl.

[24] The manufacturing method of a biological indicator according to any one of[18] to [23], wherein the discharging unit has an inlet having a filter through which the solution or the dispersion fluid is instilled.

The biological indicator of the present invention has an effect to measure if very small amount of contaminant has been removed or not with high sensitivity and to correctly evaluate effectiveness of a sterilization apparatus or the like.

According to the manufacturing method of the biological indicator of the present invention, it becomes possible to easily manufacture a biological indicator which can correctly and with high sensitivity measure degree of a sterilization apparatus or the like which carries out sterilization or the like by surface irradiation of acting radiation.

### Brief Description of the Drawings

[FIG. 1] A top view schematically showing an embodiment of a biological indicator of the present invention.
[FIG. 2] A cross-sectional view taken along A-A line of FIG. 1.
[FIG. 3] A top view schematically showing an adhering area of a biological indicator of the present invention.
[FIG. 4] An optical photomicrograph (×200) of a surface of a biological indicator of an Example 1.
[FIG. 5] An optical photomicrograph (×1000) of a surface of a biological indicator of the Example 1.
[FIG. 6] An optical photomicrograph (×200) of a surface of a biological indicator of an Example 2.
[FIG. 7] An optical photomicrograph (×1000) of a surface of a biological indicator of the Example 2.
[FIG. 8] An optical photomicrograph (×50) of a surface of a biological indicator of a comparative example 1.
[FIG. 9] An optical photomicrograph (×200) of a surface of a biological indicator of the comparative example 1.
[FIG. 10] A graph in which number of remaining bacteria is plotted to plasma irradiation time (min).
[FIG. 11] A graph showing endotoxin remaining ratio(%)after inactivation processing.

### Description of the reference numerals

1. Carrier
2. Carrier surface
3. Indicator material
3a. Contact portion
3b. Non-contact portion
5. Adhering area
6. Non-adhering area
10. Biological indicator
15. Adhering portion
16. Non-adhering portion

### The Best Mode for Carrying Out the Claimed Invention

Hereinafter, preferred embodiments of the present invention will be explained. However, the present invention is not limited to the following embodiments and it must be understood by those who are skilled in the art that any modifications or improvements are possible to the following embodiments without departing from the spirit and scope of the invention on the basis of common knowledge of those who are skilled in the art.

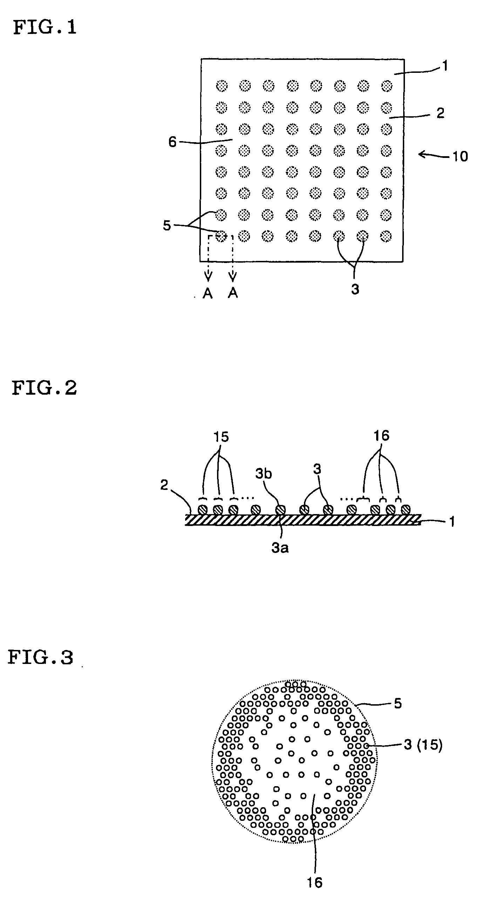

FIG. 1 is a top view schematically showing an embodiment of a biological indicator of the present invention and FIG. 2 is a cross-sectional view taken along A-A line of FIG. 1. As shown in FIGs. 1 and 2, a biological indicator 10 of the present invention includes a carrier 1 and an indicator material 3 adhering on a surface of the carrier 1 (carrier surface 2). Specific examples of the materials for the carrier 1 includes resins such as polystyrene (PS), polypropylene (PP), and polyethylene terephthalate (PET), and metals such as stainless (SUS). If the carrier 1 includes such a resin or metal, the indicator material 3 can be stably retained on the surface thereof.

The carrier 1 is normally a sheet-shaped substrate having a thickness of between 0.005 and 3 mm, preferably, between 0.01 and 1 mm. The sheet-shaped substrate is an impermeable substrate through which air, fluid, or the like cannot permeate or a permeable substrate such as a membrane through which air, fluid, or the like can permeate.

It is preferable if the carrier 1 is an impermeable substrate because the indicator material 3 to be adhering is not covered by the carrier 1 and acting radiation is irradiated to each of the indicator material 3 uniformly. Meanwhile, it is preferable if the carrier 1 is a permeable substrate such as a membrane because surface area of the carrier 1 to which the indicator material 3 adheres becomes larger and therefore overlapping of adjacent indicator materials 3 can be easily prevented. Moreover, it is preferable if suspension of the indicator material 3 is applied on the carrier surface 2, because liquid included in the suspension is quickly permeated through the carrier 1 after application and only the indicator material 3 is retained in a dispersing manner on the surface of the carrier 1.

A specific example of the indicator material 3 includes a bacterium. An example of the bacterium which can be preferably used includes a dormant spore-forming bacterium having spore form (hereinafter the "bacterium" and "spore-forming bacterium" will also be referred to as an "indicator bacterium"). A specific example of such a spore-forming bacterium includes: Geobacillus stearothermophilus, Bacillus subtilis, Bacillus atrophaeus, Bacillus subtilus. Var. niger, Bacillus pumilus, and Bacillus megaterium. These spore-forming bacteria are easy to handle and can be activated depending on the necessity and therefore are preferable. Here, among these bacteria, Geobacillus stearothermophilus and Bacillus subtilis are more preferable. Moreover, another sepcific example of the indicator material 3 includes endotoxin. If endotoxin is used as the indicator material 3, it becomes possible to correctly evaluate the effect of, especially, a toxin inactivating apparatus using plasma as acting radiation.

As shown in FIG. 2, the indicator material 3 to adhere on the carrier surface 2 has a contact portion 3a which comes into contact with the carrier surface 2 and a non-contact portion 3b which does not come into contact with the carrier surface 2. If the indicator material 3 is adhered on the carrier surface 2 in such a manner, the indicator material 3 crops out from the surface of the biological indicator 10 without fail and therefore aspect irradiated acting radiation is irradiated to most of the indicator material 3 without fail. Therefore, it becomes possible to correctly evaluate sterilization effect or the like.

As shown in FIGs. 2 and 3, the biological indicator 10 includes an adhering area 5 having an adhering portion where the indicator material 3 adheres and a non-adhering area 6 having a non-adhering portion 16 where the indicator material 3 does not adhere. Here, FIG. 3 is a top view schematically showing an example of the adhering area. Providing such adhering area 5 and the non-adhering area 6 enables to carry out evaluation with high precision of the effect of sterilization or the like using a very minute amount of the indicator material 3 and enables to carry out quantitative evaluation.

Moreover, it is preferable that a plurality of the adhering areas 5 exist as a domain (island shaped) as shown in FIG. 1. If a plurality of the adhering areas 5 exist as a domain, although there occurs distribution in adherence density of the indicator material 3 in one adhering area 5, distribution in adherence density of the indicator material 3 as a whole of the carrier 1 can be suppressed. Therefore, it becomes possible to suppress individual difference of the biological indicator and to more correctly evaluate effectiveness of the sterilization apparatus or the like.

Here, it is preferable if the outer periphery shape of the adhering area 5 as a domain is an approximately round shape. This is because the adhering area 5 can be formed on the carrier surface 2 with a high density and biological indicator itself can be more downsized. Here, the "approximately round shape" in the present application includes round shape, oval shape, elongated shape, and a partly distorted round shape. Moreover, as shown in FIG. 3, it is preferable that the density of the indicator material 3 (adhering density) in the adhering area 5 becomes higher in the outer periphery portion of the adhering area 5 and in the vicinity thereof. If the adhering density of the indicator material 3 becomes higher in the outer periphery portion of the adhering area 5 and in the vicinity thereof, it becomes easier to recognize the adhering condition of the indicator material 3 as an approximately round shaped one and therefore it becomes easier to check adhering condition of the indicator material 3 when a biological indicator is manufactured.

It is preferable that all of the indicator materials 3 exist within a range of 2 µm from the carrier surface 2 (refer to FIG. 2). If the indicator material 3 is adhered to the carrier 1 so that all the indicator materials 3 exist within the above-mentioned range, the aspect irradiated acting radiation is irradiated to almost all of the indicator materials 3 without fail. Therefore, it becomes possible to more correctly evaluate the effect of the acting radiation. Moreover, if the indicator material 3 is an endotoxin, it is preferable that all of the indicator materials 3 exist within a range of 100 nm from the carrier surface 2. It is more preferable if the indicator materials 3 exist within a range of 50 nm from the carrier surface 2 and it is further more preferable if the indicator materials 3 exist within a range of 10 nm from the carrier surface 2. If the endotoxin is adhered to the carrier 1 so that the endotoxin can exist within the above-mentioned range, such a condition sufficiently simulates practical contamination condition by endotoxin and the aspect irradiated acting radiation is irradiated to almost all of the indicator materials 3. Therefore, it becomes possible to more correctly evaluate inactivation effect of the acting radiation to the endotoxin.

It is preferable that the number of indicator bacterium is "between 5 × 10⁵ and 2 × 10⁶. It becomes possible to correctly evaluate sterilization effect of a sterilization apparatus or the like at a level closer to practical usage if the number of indicator bacterium adhered on the carrier surface 2 is set within the above-mentioned range. Here, if the number of the indicator bacterium is less than 5 × 10⁵, because the number of the indicator bacterium is too small, there may be a case where evaluation cannot be carried out sufficiently. On the other hand, if the number of the indicator bacterium exceeds 2 × 10⁶, there is a possibility that it is misjudged that there is "no effect" in case sterilization apparatus or the like practically having sterilization ability is used.

Moreover, it is preferable that the number of the indicator bacterium adhering in one adhering area 5 as a domain is between 1 and 100 and it is more preferable if the number is between 1 and 10. If the number of the indicator bacterium adhering in one adhering area 5 as a domain exceeds 100, the adhering area 5 must be expanded so that the indicator bacteria do not overlap in one adhering area 5 and therefore, there is a tendency that large density distribution easily occurs. If the number of the indicator bacterium adhering in one adhering area 5 as a domain is 10 or less, less number of indicator bacteria overlap to each other and therefore it is preferable.

It is preferable that at least a part of 90% or more of the indicator bacteria can be visually confirmed when seen from a direction perpendicular to the carrier surface 2 (when seen from the upper direction of the page of FIG. 1). Moreover, it is preferable if adjacent indicator bacteria adhere on the surface of the carrier 1 without overlapping to each other. If the adhering condition of the indicator bacteria is set as above, even in a case where the acting radiation is aspect irradiated in a linear fashion, the acting radiation is irradiated to more indicator bacteria and therefore it becomes possible to more correctly evaluate the effectiveness of the sterilization apparatus or the like. Here, it is more preferable if ratio of the indicator bacteria at least part of which can be visually confirmed when seen from a direction perpendicular to the carrier surface 2 is 95% or more. It is especially preferable if the ratio is 99% and most preferable if the ratio is 100%.

It is preferable that at least protein or polymer exists on the surface of the carrier 1. With the existence of protein or polymer, it becomes possible to disperse the indicator bacteria on the carrier surface 2 and to cause them adhered in a stable condition with small amount of bacteria being dropped out while there is substantially no effect to the sterilization or the like. Here, a preferable example of the protein includes albumin. Moreover, a preferable example of the polymer includes carboxylmethyl cellulose, polyethylene glycol, and the like.

Because the biological indicator of the present invention includes the above-mentioned configuration, the biological indicator can be preferably used as an indicator for evaluation of effectiveness of a disinfection, sterilization, bacteria elimination, or toxin degradation apparatus using radiation for disinfection, sterilization, bacteria elimination, or toxin degradation (acting radiation) which is aspect irradiated to a target. Here, a specific example of the acting radiation to be aspect irradiated includes aspect irradiation type plasma, ultraviolet, electron ray, and the like.

Next, an embodiment of a manufacturing method of a biological indicator of the present invention will be explained. To manufacture a biological indicator of the present invention, solution or dispersion fluid including an indicator material is prepared first. Solvent, or liquid for preparation of the solution or the dispersion fluid includes, for example, water, alcohol liquid, and the like. Here, a specific example of alcohol included in the alcohol liquid includes methanol, ethanol, isopropyl alcohol, and the like. The prepared solution or the dispersion fluid is discharged as minute droplets from a discharging unit by ink jet method so that the droplets land on the surface of a predetermined carrier. An ink jet discharging unit to be preferably used includes, for example, an ink jet spotter or the like disclosed in JP-A-2001-124789.

If the ink jet spotter or the like is used, the solution or dispersion fluid in which the indicator material is solved or dispersed can be made into a state of minute droplets which are controlled to a certain amount by pl order and the droplets can be discharged uniformly on the surface of the carrier in a plurality of times by several hundreds of thousands of shots. Therefore, it becomes possible to cause the indicator material to be uniformly adhered on the surface of the carrier. Moreover, if dispersion fluid in which a solid substance such as indicator bacteria is dispersed is discharged in a minute droplet form by use of the ink jet discharging unit, because the indicator bacteria included in the minute droplet are dispersed by the force generated when the droplet lands on the surface, it becomes possible to distribute and attach the indicator bacteria on the carrier surface while avoiding overlapping of the adjacent indicator bacteria.

Here, if a permeable substrate such as a membrane is used as a carrier, the indicator bacterium which is solid particle remains on a portion exposed to the surface of fiber included in the permeable substrate and the liquid is quickly absorbed inside the fiber. Therefore, the indicator bacteria included in the minute droplet which landed on the surface of the carrier are not spread too much and are adhered on the surface of the carrier while preventing overlapping. Moreover, since the indicator bacteria included in the minute droplet are not spread too much, the discharged minute droplets can be positioned in the vicinity of each other. Therefore, it becomes possible to improve density of the indicator bacteria which can be adhered on the whole of the carrier.

It is preferable that the quantity of a drop of the minute droplet discharged from the discharging unit is between 1 and 500 pl. It is more preferable if the quantity is between 10 and 300 pl and is especially preferable if the quantity is between 50 and 200 pl. If the quantity of a drop of the minute droplet is set within the above-mentioned quantity, it becomes possible to discharge the droplets more uniformly on the whole surface of the carrier. Here, if the quantity of a drop of the minute droplet is less than 1 pl, there may be a case where the indicator material is not included in a drop of the minute droplet. On the other hand, if the quantity of a drop of the minute droplet is more than 500 pl, because too much quantity of liquid is discharged at once, there is a tendency that discharging is unstably carried out as amplitude at liquid surface at the discharging nozzle of the discharging unit (meniscus) becomes large, and liquid leaks out from the discharging nozle.

It is preferable that bacteria (indicator bacteria) are dispersed with the concentration of between 1 × 10² and 1 × 10⁷/µl in the dispersion fluid. It is more preferable if the concentration is between 1 × 10³ and 1 × 10⁶/µl and is especially preferable if the concentration is between 1 × 10⁴ and 1 × 10⁶/µl. If the concentration of the indicator bacteria included in the dispersion fluid exceeds 1 × 10⁷/ µl, there may be a case where dispersiveness of the indicator bacteria on the carrier surface is lowered and density of the indicator bacteria to adhere on the surface is fluctuated. On the other hand, if the concentration of the indicator bacteria included in the dispersion fluid is less than 1 × 10²/µl, while dispersiveness of the indicator bacteria on the carrier surface is fine, quantity of dispersion fluid to be discharged becomes large and therefore there is a tendency that production cost rises. Here, in case the indicator bacterium is a spore-forming bacterium and dispersion fluid having the spore-forming bacteria within a range of the above-mentioned concentration (5 × 10⁴/µl) is discharged as minute droplets having the quantity within the above-mentioned range (between 50 and 200 pl), the number of the spore-forming bacteria included in a drop of the minute droplet can be set to between 2 and 10. Thus, adjacent spore-forming bacteria hardly overlap with each other and many spore-forming bacteria can be caused to uniformly adhere on the carrier surface.

It is preferable that the dispersion fluid is alcohol dispersion fluid in which the indicator bacteria are dispersed in alcohol liquid. In such alcohol dispersion fluid, the indicator bacteria do not easily sink in the discharging unit after elapse of several hours. Therefore, troubles such as ratio of the indicator bacteria included in the minute droplet discharged from the discharging unit changes while discharging or the discharging hole (nozzle) is clogged hardly occur.

Moreover, it is preferable that the alcohol liquid to disperse the indicator bacteria is alcohol aqueous solution having concentration of alcohol such as methanol of between 20 and 80% by mass and it is more preferable if the solvent is alcohol solution having between 30 and 60% by mass of alcohol concentration. If the concentration of alcohol is less than 20% by mass, there may be a case where dispersiveness of the indicator bacteria is lowered. On the other hand, if the concentration of alcohol exceeds 80% by mass, amount of evaporation during discharging operation is easy to increase and there may be a case where the concentration of the indicator bacteria becomes nonuniform. Moreover, required amount of alcohol is increased and there may be a disadvantage in view of manufacturing cost.

Here, in case if the indicator bacterium is spore-forming bacterium and liquid to disperse the indicator bacterium is alcohol liquid, it is preferable that ratio of the spore-forming bacteria included in the dispersion fluid is between 1 × 10³/ µl and 1 × 10⁷/µl. It is more preferable if the ratio is between 1 × 10⁴/µl and 1 × 10⁶/µl and is especially preferable if the ratio is between 1 × 10⁵/µl and 1 × 10⁶/ µl. At the same time, it is preferable that the quantity of a drop of the minute droplet to be discharged is between 50 and 200 pl. The alcohol liquid has superior dispersiveness of the spore-forming bacteria in the liquid and therefore it is possible to discharge the fluid even with higher concentration without causing clogging in the discharging unit.

Here, it is preferable that the discharging unit for discharging the solution or dispersion fluid has a filter at an inlet from which the solution or dispersion fluid is instilled because the filter removes impurity such as dust included in the solution or dispersion fluid and discharging can be carried out more surely. Moreover, In case of discharging dispersion fluid in which indicator bacteria are dispersed, using a discharging unit having such an inlet enables to disperse indicator bacteria which are easy to agglutinate. Therefore, it becomes possible to more easily prevent indicator bacteria which are adjacent on the carrier surface from overlapping to each other. Here, in case of discharging dispersion fluid in which indicator bacteria are dispersed, it is required for a micropore diameter of the filter to be large enough to prevent clogging of the indicator bacteria.

After the minute droplet discharged from the discharging unit lands on the surface of a carrier, the landed minute droplet is dried to obtain a biological indicator of the present invention. A method to dry the minute droplet is not especially limited. However, the drying may be carried out by air drying or by drying in an appropriately heated condition.

### Examples

Hereinafter, the present invention will be explained more in detail on the basis of embodiments. However, the present invention is not limited to these embodiments.

[Sporogenous dispersion fluid (1)]: Product name "Bacterial Spore Suspension" (10E-6, G. stearothermophilus [ATCC7953], number of bacteria 1 × 10⁶/0.1ml, dispersed in water) of Raven Labs was used as "sporogenous dispersion fluid (1)".

[Sporogenous dispersion fluid (2)]: Product name "Spordex Suspension" (10E-6, G. stearothermophilus [ATCC7953], number of bacteria 2.9 × 10⁶/0.1ml, dispersed in 40% methanol solution) of Steris Corporation was used as "sporogenous dispersion fluid (2)".

[Endotoxin solution]: Product name "L2630" (derived from E. coli (O111 strain)) of Sigma was dissolved by endotoxin free water to prepare solution with concentration of 1.5 mg/ml. The solution was used as "endotoxin solution".

[Carrier (1)]: A PP sheet (Product of Futamura Chemical Co., Ltd., product class "FHK2", count "50", dimension: 70mm × 70 mm × 0.05 mm) processed by following plasma processing condition was used as "Carrier (1)".

[Carrier (2)]: A PS sheet (Product of Ooishi Sangyo, product name "OSK-Styrofan-30S-C", dimension: 15 mm × 15 mm × 0.03 mm) processed by following plasma processing condition was used as "Carrier (2)".

<Plasma processing condition>
Input power: 16.0mJ/p
Vp: 19.5kV
Ip: 15.6A
Frequency: 2.5kHz
Temp: 60 to 65°C
Area: 300 × 300 mm²
Gap: 40 mm
N₂ flow speed: 6L/min
Pressure: 45kPa

### (Example 1)

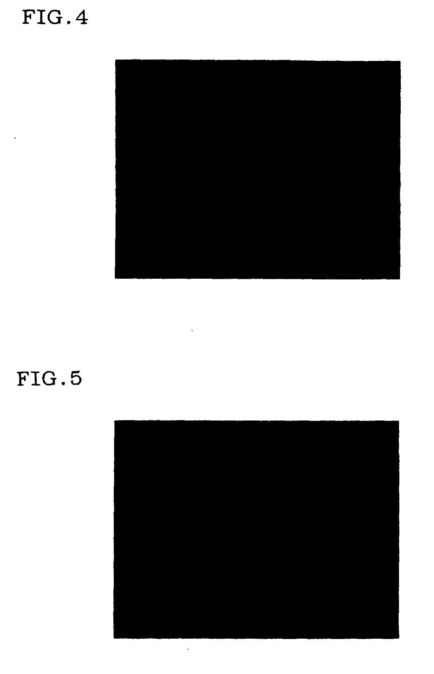

The sporogenous dispersion fluid (1) was concentrated tenfold and mixed with the same quantity of albumin solution (albumin concentration 2mg/ml) to obtain dispersion fluid for discharging (number of bacteria: 5 × 10⁴/µl, albumin concentration 1mg/ml). By use of an ink jet spotter disclosed in JP-A-2001-124789, the dispersion fluid for discharging thus obtained was spotted on the surface of the carrier (1). Quantity in one spot was 200 pl, the number of bacteria included in one spot was 10, pitch was 200 µm, and number of spots was 100,200 (334 × 300) for the spotting. The dispersion fluid thus spotted was dried to obtain a biological indicator (Example 1). Number of bacteria per one sheet of the biological indicator thus obtained was 1 × 10⁶. Moreover, optical photomicrographs of a surface of the biological indicator thus obtained are shown in FIGs. 4 (×200) and 5 (×1000).

### (Example 2)

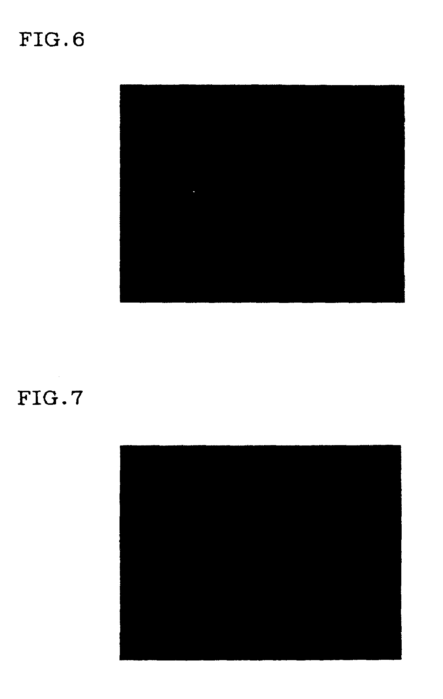

The sporogenous dispersion fluid (2) was concentrated tenfold to obtain dispersion fluid for discharging (number of bacteria: 2.9 × 10⁵/µl, dispersed in 40% by mass of methanol solution). By use of an ink jet spotter disclosed in JP-A-2001-124789, the dispersion fluid for discharging thus obtained was spotted on the surface of the carrier (1). Quantity in one spot was 100 pl, number of bacteria included in one spot was 29, pitch was 100 µm, and number of spots was 42,000 (600 × 700) for the spotting. The dispersion fluid thus spotted was dried to obtain a biological indicator (Example 2). Number of bacteria per one sheet of the biological indicator thus obtained was 1.218 × 10⁶. Moreover, optical photomicrographs of a surface of the biological indicator thus obtained are shown in FIGs. 6 (× 200) and 7 (X1000).

### (Comparative Example 1)

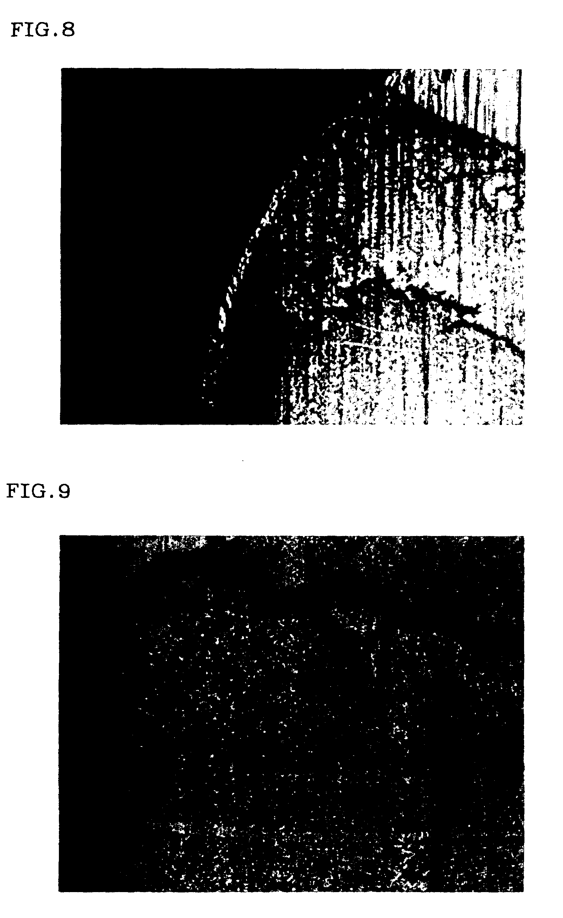

100µm of sporogenous dispersion fluid(1) was dropped on a surface of the carrier (1) by use of a dropper and was dried to obtain a biological indicator (Comparative Example 1). Number of bacteria per one sheet of the biological indicator was 1 × 10⁶. Moreover, optical photomicrographs of a surface of the biological indicator thus obtained are shown in FIGs. 8 (×50) and 9 (×200).

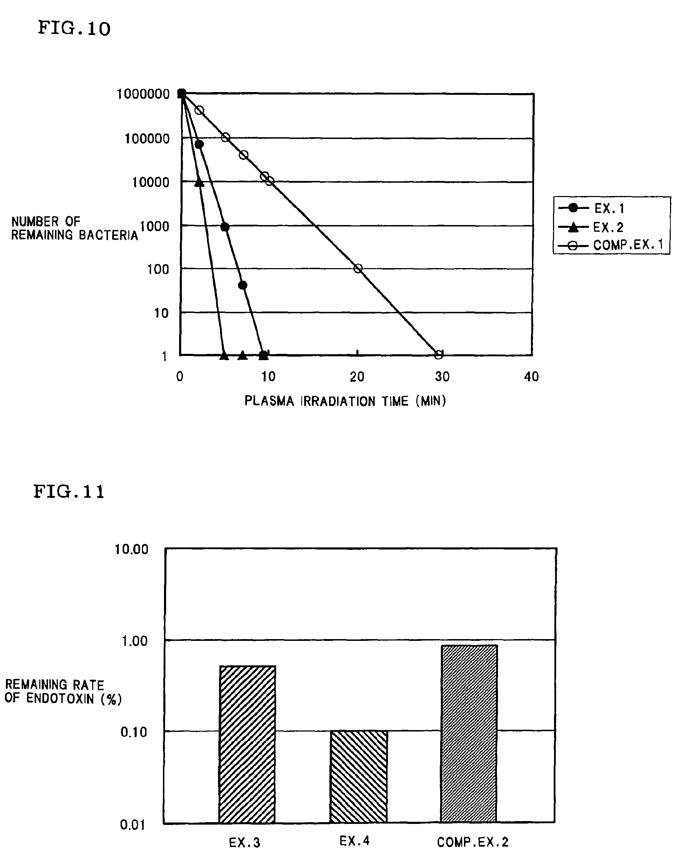

[Evaluation (1)]: By use of a TES sterilization apparatus described in the application of JP-A-2005-310196, biological indicators of the embodiments 1 and 2 and the comparative example 1 were processed by the following sterilization condition. The biological indicators taken out from the sterilization apparatus were dipped in sterilization water and the sterilization water diluted appropriately was poured on an SCD agar medium (product of Kanto Chemical Co., Inc., code "CM 0129") for 48-hour cultivation at the temperature of 58°C. Then, number of the remaining bacteria was counted. Results are shown in Table 1. Moreover, a graph in which number of remaining bacteria is plotted to plasma irradiation time (min) is shown in FIG. 10.

### <Sterilization condition>

Sterilization pulse power source: IES power source (K. Iida and K. Sakuma, "Nanopulse generator employing a SI. thyristor (IES circuit)", the 15th Symposium of Static Induction Devices, ISSN1340-5853, SSID-02-9, pp. 45-50 (2002))
Pulse application condition: Pulse half value of width is 200ns Distance between cathode (sample) and anode: 40 mm Processing procedure: A biological indicator is provided on a cathode inside the sterilization apparatus to be plasma irradiated. The biological indicator is taken out after a predetermined period of processing time.

**[Table 1]**

| | | Plasma irradiation time (min) | | | | | | | |
|---|---|---|---|---|---|---|---|---|---|
| | | 0 | 2 | 5 | 7 | 9.5 | 10 | 20 | 29.5 |
| Example 1 | Number of remaining bacteria | 980000 | 70000 | 900 | 41 | 1 | - | - | - |
| Example 2 | | 980000 | 9800 | 1 | 1 | 1 | - | - | - |
| Comparative Example 1 | | 980000 | 400000 | 100000 | 40000 | 13000 | 10000 | 100 | 1 |

### (Consideration)

It is obvious from the results shown in Table 1 and FIG. 10 that indicator bacteria disappeared (were sterilized) by shorter plasma irradiation time in the biological indicators of the embodiments 1 and 2 compared to the comparative example 1. Here, as shown in FIGs. 8 and 9, in the biological indicator of the Comparative Example 1, a portion where the indicator bacteria are overlapped is shown in deep gray. On the other hand, as shown in FIGs. 4 to 7, the indicator bacteria do not overlap in the biological indicators of the embodiments 1 and 2 and are equally distributed.

### (Example 3)

By use of an ink jet spotter disclosed in JP-A-2001-124789, endotoxin solution was spotted on the surface of the carrier (2). Quantity in one spot was 100 pl, quantity of endotoxin included in one spot was 150 pg, pitch was 1000 µm, and number of spots was 100 (10 × 10) for the spotting. The endotoxin solution thus spotted was dried to obtain a biological indicator (Example 3). Quantity of endotoxin per one sheet of the biological indicator thus obtained was 15 ng.

### (Example 4)

By use of an ink jet spotter disclosed in JP-A-2001-124789, endotoxin solution was spotted on the surface of the carrier (2). Quantity in one spot was 100 pl, quantity of endotoxin included in one spot was 150 pg, pitch was 500 µm, and number of spots was 400 (20 × 20) for the spotting. The endotoxin solution thus spotted was dried to obtain a biological indicator (Example 4). Quantity of endotoxin per one sheet of the biological indicator thus obtained was 60 ng.

### (Comparative Example 2)

Endotoxin solution was dropped on a surface of the carrier (2) by use of a dropper and was dried to obtain a biological indicator (comparative example 2).

### [Evaluation 2]

By use of a TES sterilization apparatus described in the application of JP-A-2005-310196, biological indicators of the embodiments 3 and 4 and the comparative example 2 were processed by the following inactivation condition. The biological indicators taken out from the sterilization apparatus were dipped in endotoxin free water. Then, ultrasonic wave processing was carried out to the biological indicators for 15 minutes. 0.1 ml of water after ultrasonic wave processing was taken to measure collected quantity of endotoxin by reagent exclusively for measuring endotoxin (product name "Limulus ES-II Test Wako", Wako Pure Chemical Industries, Ltd.) and an endotoxin measuring apparatus (product name "Toxinometer-ET-2000", Wako Pure Chemical Industries, Ltd.). Results are shown in Table 2. Moreover, a graph showing remaining rate (%) of endotoxin after inactivation processing is shown in FIG. 11.

### <Inactivation condition>

Sterilization pulse power source: IES power source (K. Iida and K. Sakuma, "Nanopulse generator employing a SI. thyristor (IES circuit)", the 15th Symposium of Static Induction Devices, ISSN1340-5853, SSID-02-9, pp. 45-50 (2002))
Pulse application condition: Pulse half value of width is 200 ns
Distance between cathode (sample) and anode: 40 mm Processing procedure: A biological indicator is provided on a cathode inside the sterilization apparatus to be plasma irradiated. The biological indicator is taken out after 15 minutes of processing time.

**[Table 2]**

| | Endotoxin solution concentration (mg/ml) | Adhering method | Number of spots | Adhering quantity (ng) | Endotoxin | | |
|---|---|---|---|---|---|---|---|
| | | | | | Collected quantity (ng) | | Remaining rate (%) |
| | | | | | Before inactivation processing | After inactivation processing | |
| Example 3 | 1.5 | Ink jet | 100 | 15 | 15 | 0.076 | 0.51 |
| Example 4 | 1.5 | Ink jet | 400 | 60 | 61 | 0.059 | 0.10 |
| Comparative Example 2 | 1.5 | Dropped from dropper | 1 | - | 2 | 0.017 | 0.85 |

### (Consideration)

It is obvious from the results shown in Table 2 and FIG. 11 that by the biological indicators of the embodiments 3 and 4, endotoxin is dissolved in a shorter period under plasma irradiation compared to the biological indicator of the Comparative Example 2.

### Industrial Applicability

The biological indicator of the present invention can be preferably used as an indicator for evaluating effectiveness of a sterilization apparatus or the like which carries out sterilization or the like by surface irradiating acting radiation.

## Claims

1. A biological indicator including:
a carrier, and
an indicator material; said indicator material adhering on the surface of the carrier, and having a contact portion being contact with the surface of the carrier and a non-contact portion being not in contact with the surface of the carrier, and
wherein the carrier includes an adhering area having a adhering portion to which the indicator material adheres and a non-adhering area having an non-adhering portion to which the indicator material does not adhere.

2. The biological indicator according to claim 1,
wherein all of the indicator materials exist within a range of 2 µm from the carrier surface.

3. The biological indicator according to claim 1 or 2,
wherein a plurality of the adhering areas exist as a domain.

4. The biological indicator according to claim 3,
wherein the shape of the plurality of adhering areas existing as a domain is approximately circular form, and density of the indicator materials in each of the plurality of adhering areas becomes higher in an outer circumference portion and in the vicinity thereof of the adhering area.

5. The biological indicator according to any one of claims 1 to 4,
wherein the indicator material is a spore-forming bacterium.

6. The biological indicator according to claim 5,
wherein at least a part of 90% or more of the spore-forming bacteria can be visually confirmed when seen from a direction perpendicular to the surface of the carrier.

7. The biological indicator according to claim 6,
wherein adjacent spore-forming bacteria adhere on the carrier surface without overlapping to each other.

8. The biological indicator according to claims 6 or 7,
wherein a plurality of the adhering areas exist as a domain, and at the same time 1 to 100 spore-forming bacteria are included in one adhering area.

9. The biological indicator according to any one of claims 5 to 8,
wherein at least protein or polymer exists on the carrier surface.

10. The biological indicator according to claim 9,
wherein the protein is albumin.

11. The biological indicator according to claim 9,
wherein the polymer is carboxyl methyl cellulose or polyethylene glycol.

12. The biological indicator according to any one of claims 5 to 11,
wherein the number of spore-forming bacteria adhering on the carrier surface is between 5 × 10⁵ and 2 × 10⁶.

13. The biological indicator according to any one of claims 1 to 4,
wherein the indicator material is endotoxin.

14. The biological indicator according to claim 13,
wherein all of the indicator materials exist within a range of 100 nm from the carrier surface.

15. The biological indicator according to any one of claims 1 to 14,
wherein the carrier is an impermeable substrate through which fluid cannot permeate or a permeable substrate through which fluid can permeate.

16. The biological indicator according to any one of claims 1 to 15,
wherein the material constituting the carrier is one member selected from the group consisting of polystyrene, polypropylene, polyethylene terephthalate, or stainless.

17. The biological indicator according to any one of claims 1 to 16, which is to be used to evaluate effectiveness of a disinfection, sterilization, bacteria elimination, or toxin degradation apparatus using radiation for disinfection, sterilization, bacteria elimination, or toxin degradation which is aspect irradiated to a target.

18. A method for manufacturing a biological indicator according to any one of claims 1 to 17 comprising:
a step of discharging solution or dispersion fluid including an indicator material from a discharging unit as minute droplets by ink jet method so that the droplets are caused to land on a carrier surface and thereafter drying the droplets which have landed on the carrier surface.

19. The manufacturing method of a biological indicator according to claim 18,
wherein the quantity of a drop of the minute droplet is between 1 and 500 pl.

20. The manufacturing method of a biological indicator according to claim 18 or 19,
wherein the indicator material is a spore-forming bacterium, and the dispersion fluid includes the spore-forming bacterium dispersed with the concentration of 1 ×10² to 1 × 10⁷/µl.

21. The manufacturing method of a biological indicator according to claim 20,
wherein the dispersion fluid is alcohol dispersion fluid in which the spore-forming bacteria are dispersed in an alcohol solvent.

22. The manufacturing method of a biological indicator according to claim 21,
wherein the alcohol solvent is alcohol aqueous solution having alcohol concentration of between 20 and 80% by mass.

23. The manufacturing method of a biological indicator according to any one of claims 20 to 22,
wherein the dispersion fluid is the spore-forming bacteria dispersed into the concentration of 1 × 10³ to 1 × 10⁷/ µl, and the quantity of a drop of the minute droplet is between 50 and 200 pl.

24. The manufacturing method of a biological indicator according to any one of claims 18 to 23,
wherein the discharging unit has an inlet having a filter through which the solution or the dispersion fluid is instilled.
